Europäisches Patentamt

European Patent Office

Office européen des brevets

(19)

(11) Publication number: **0 527 891 B1**

# EUROPEAN PATENT SPECIFICATION

(45) Date of publication of patent specification: **26.07.95**  (51) Int. Cl.⁶: **A61K  9/52**

(21) Application number: **91909670.1**

(22) Date of filing: **01.05.91**

(86) International application number:
**PCT/US91/02842**

(87) International publication number:
**WO 91/16887 (14.11.91 91/26)**

(54) **BIODEGRADABLE POLYESTERS FOR SUSTAINED DRUG DELIVERY.**

(30) Priority: **01.05.90 US 517340**

(43) Date of publication of application:
**24.02.93 Bulletin  93/08**

(45) Publication of the grant of the patent:
**26.07.95 Bulletin  95/30**

(84) Designated Contracting States:
**AT BE CH DE DK ES FR GB GR IT LI LU NL SE**

(56) References cited:
**EP-A- 0 334 062**
**WO-A-89/05664**
**US-A- 4 148 871**
**US-A- 4 702 917**
**US-A- 4 863 472**

(73) Proprietor: **RESEARCH TRIANGLE INSTITUTE**
**Cornwallis Road, Hanes Building**
**Research Triangle Park, NC 27709 (US)**

(72) Inventor: **SCHINDLER, Anton**
**3742 Bentley Drive**
**Durham, NC 27707 (US)**
Inventor: **HOLLOMON, Martha, G. 115 Charter**
**Courter**
**Colonial Townes**
**Cary, NC 27511-5095 (US)**

(74) Representative: **Des Termes, Monique et al**
**c/o Société de Protection des Inventions**
**25, rue de Ponthieu**
**F-75008 Paris (FR)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 527 891 B1

**Description**

Technical Field

The present invention relates to novel polymer-drug formulations possessing improved drug permeabilities. More specifically, this invention relates to random copolymers of caprolactone and trimethylene carbonate (1.3-dioxane-2-one) possessing high molecular weight, and the use of said copolymers for the fabrication of a biodegradable, tubular device in the form of a cylindrical capsule useful for the sustained delivery of drugs after subcutaneous implantation of said device in humans or animals.

Background Art

U.S. Patents 4,243,775 and 4,300,565 to Rosensaft et al disclose a procedure of sequential monomer addition to form partially ordered copolymers composed predominantly of glycolide in combination with other cyclic ester monomers such as trimethylene carbonate to be used for the fabrication of surgical articles, particularly sutures. U.S. Patent 4,429,080 to Casey et al discloses the fabrication of triblock copolymers possessing trimethylene carbonate center blocks to be used for the production of surgical articles, particularly sutures and ligatures. U.S. Patents No. 4,788,979 and 4,791,929 to Jarrett et al disclose the production of low molecular weight copolymers of caprolactone and trimethylene carbonate to be useful as bioabsorbable coatings of surgical articles such as sutures. All of these patents do not, however, disclose the concept of using random copolymers of high molecular weight to provide drug formulation for the sustained subdermal delivery of drugs.

U.S. Patent No. 4,503,216 to Fagerburg et al discloses the reaction of poly(caprolactone) with ethylene carbonate to produce difunctional hydroxyl-terminated polyether-esters useful as liquid urethane forming polyols. U.S. Patent 4,810,775 to Bendix et al discloses a process relating to the purification of polyesters, particularly resorbable polyesters, by precipitating the polymers under the effect of high shear forces. Both patents do not disclose the concept of sustained drug delivery devices.

U.S. Patent 3,887,699 to Yolles discloses a device for dispersing drugs from a biodegradable polymeric material which is shown to be polylactate. U.S. Patent 4,148,871 to Pitt et al discloses the use of biodegradable homopolymers and copolymers of caprolactone for formulating devices to accomplish the sustained subdermal delivery of drugs. The patent does not disclose the fabrication or the use of devices incorporating trimethylene carbonate.

U.S. Patent No. 4,702,917 to Schindler discloses the use of homopolymers of caprolactone and of its copolymers with other lactones in combination with aliphatic polyethers to formulate tubular drug delivery devices possessing walls of defined porosity, particularly useful for the delivery of hydrophilic compounds such as polypeptides. The patent does not disclose the fabrication or the use of devices which incorporate trimethylene carbonate and which possess a dense, non-porous wall.

Disclosure of the Invention

The present invention relates to biodegradable, sustained drug delivery devices for subcutaneous implantation, said devices being fabricated from random copolymers of caprolactone and trimethylene carbonate inside a narrow compositional range, and said devices providing higher drug release rates than obtainable with similar devices described in the prior art. The higher drug release rates achieved with devices of this invention permits the use of smaller devices than possible according to the prior art, said devices still providing the required therapeutical levels of released drug but with a concomitant improvement in comfort to the patient and a reduction in the body burden during bioadsorption of said device after exhaustion of the contained drug. It was found that cylindrical, tubular devices, possessing good mechanical strength and formstability, could be produced from a random copolymer containing trimethylene carbonate and caprolactone in a mole ratio of about 1:5, said devices exhibiting drug release rates for levonorgestrel being three times the drug release rates of polycaprolactone devices of identical geometric form and dimension.

The present invention addresses the formulation of a bioabsorbable pharmaceutical device for subdermal administration, designed to release effective amounts of drug during a predetermined time period. The drug delivery device of this invention is composed of a drug core surrounded by a polymer shell in the form of a short, cylindrical tube with both ends sealed to form a capsule which can be administered by means of a trocar. The term drug is therein to be understood as defined in the Federal Food, Drug, and Cosmetic Act, Section 201(2)g. Specifically mentioned drugs may include: steroidal and nonsteroidal contraceptive agents, male and female hormones, narcotics and narcotic antagonists, as well as antineoplastic and antiinflammatory agents. The dense, non-porous structure of the capsule wall requires drugs which are soluble to some extent in the polymer wall and excludes most drugs of strongly hydrophilic nature.

The drug delivery device of the present invention belongs to the category of implantable reservoir devices, a category which encompasses devices where the drug is contained inside a surrounding polymer shell which represents the reservoir and acts as a barrier controlling the release of the drug from the implanted device into the surrounding tissue. The rate at which the drug is released from the device is governed by the permeability of the polymer shell for the drug under consideration which, in turn, depends on the product of two material constants characteristic for a given polymer-drug combination, namely, the diffusivity and the solubility of the drug in the polymer it is combined with. Both material constants are strongly affected by the morphology of the polymer, particularly its degree of crystallinity, insofar as both the dissolution of the drug in the polymer shell and its diffusive transport through the polymer shell take place exclusively in the amorphous phase of the polymer. Consequently, a decrease of the crystallinity of the polymer causes an increase of both the solubility and the diffusivity of the drug, resulting in an increase of the permeability of the device and a correspondingly higher drug delivery rate.

In an alternative embodiment, an implantable sustained delivery device may comprise the copolymer in matrix form, defining an intimate mixture of drug and copolymer. Preferably, such implants are cylindrical or spherical in shape.

The desirable increase of the drug permeability of a reservoir device attained by decreasing the crystallinity of its polymeric shell finds its limitation in the loss of formstability of the device accompanying low crystallinity contents. Polymers useful as reservoir devices for sustained drug delivery systems need to have glass transition temperatures below body temperature to assure sufficiently high diffusivity and solubility of the drugs. The formstability of such devices is then assured by the presence of physical crosslinks in form of crystalline domains; with excessive loss of the latter the formstability is lost too.

## Brief Description of the Invention

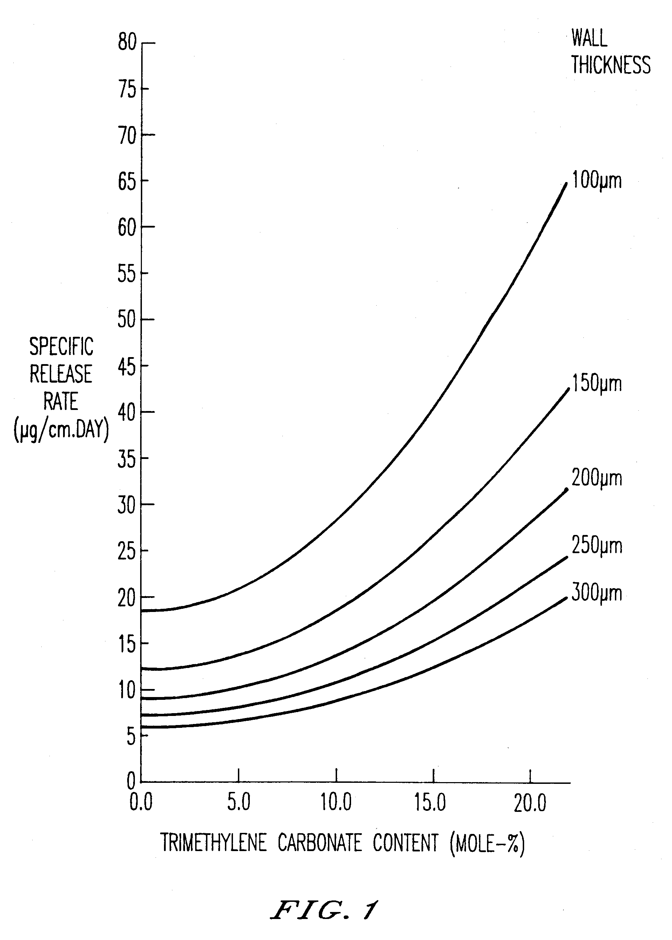

The Figure illustrates the effect of the trimethylene carbonate content of copolymers with caprolactone on the specific release rate of levonorgestrel (in $\mu$g/cm.day) from cylindrical capsules fabricated from these copolymers and possessing 2.4 mm outer diameter and different wall thicknesses in the range of 0.1-0.3 mm.

## Best Mode For Carrying Out The Invention

According to the present invention, the polymer shell of the device consists of a random copolymer of caprolactone with trimethylene carbonate containing not less than 5 and not exceeding 25 mole-% of trimethylene carbonate, the preferred compositional range being 10-20 mole-%. In this embodiment of the invention the term random copolymer means the result of a copolymerization reaction utilizing intimately mixed comonomers by charging into the reactor a solution of trimethylene carbonate in caprolactone. Under this condition a random distribution of both comonomer units in the polymer chain results although variations in reaction conditions can affect to some extent the degree of randomness of the comonomer distribution.

Surprisingly, optimal properties concerning the required combination of formstability, mechanical strength, and drug permeability are observed only with drug delivery devices fabricated from copolymers with compositions inside the narrow range of 10-20 mole-% trimethylene carbonate contents. Devices utilizing copolymers with compositions outside the indicated range of 10-20 mole-% trimethylene carbonate contents are deficient in some of the properties in comparison to copolymers with compositions inside the indicated optimal range. Copolymers containing less than 10 mole-% trimethylene carbonate, although possessing good mechanical properties, are limited in their drug permeabilities which do not exceed that of polycaprolactone by more than 50%. Copolymers with trimethylene carbonate values exceeding the optimal compositional range are of increasingly lower crystallinities and, consequently, lack the required formstability. Indeed, the formstability of devices fabricated from copolymers exceeding a trimethylene carbonate content of 25 mole-% is so low to preclude permeability determinations to be performed.

In Table 1 are summarized mechanical properties of three copolymers, COP-I-, -11 and -12, possessing trimethylene carbonate contents of 11.3, 16.5 and 21.9 mole-%, respectively, together with the corresponding properties of polycaprolactone, designated PCL, for comparison. The data were obtained at ambient temperature on compression molded films of about 0.3 mm thickness utilizing an Instron Universal Testing Machine, Model 112. Inside the compositional range of interest all of the determined mechanical properties of the copolymers were found to be linearly dependent on their trimethylene carbonate contents. The relevant linear relations are as follows wherein the expression (TMC) represents the trimethylene carbonate content of the copolymers expressed in mole-%:

$$Young's\ Modulus\ (MP2) = 259.1 - 8.18\ (TMC)$$
$$Yield\ Stress\ (MP2) = 16.2 - 0.522\ (TMC)$$
$$Nominal\ Stress\ at\ Break\ (MP2) = 30.7 - 0.605\ (TMC)$$
$$Draw\ Ratio\ at\ Break = 8.22 + 0.557\ (TMC)$$

Inside the optimal compositional range, the caprolactone-trimethylene carbonate copolymers combined a surprisingly high tensile strength with exceptionally high draw ratios. This combination of properties is especially advantageous for the intended use of the copolymers as subcutaneous drug delivery implants since these properties reduce the likelihood of rupture of a device during use or during surgical removal if indicated.

In Table 2 are presented the crystallinity contents and the melting points of caprolactone-trimethylene carbonate copolymers together with the permeabilities for levonorgestrel of drug delivery devices fabricated from several of these copolymers including polycaprolactone. Crystallinity contents and melting points were determined by differential scanning calorimetry utilizing Perkin-Elmer equipment, Model DSC-2 with thermal data station. The permeability values for levonorgestrel were derived from in vitro drug release studies performed in water at 37.5°C Polymer tubes of about 2.0 cm length, 2.1 2.6 mm outer diameter, and wall thicknesses in the range of 0.1-0.3 mm were filled with about 15 - 20 mg dry, micronized drug and heat sealed at both ends. The resulting capsules were kept submerged in 200 ml water contained in 500 ml Erlenmeyer flasks, thermostated at 37°C under mild rotary shaking. The released drug was monitored spectrophotometrically at 240 nm.

The permeabilities of copolymers of different compositions for levonorgestrel were derived from measurements of the steady state release rates and device dimensions. The steady state release of a drug from a cylindrical reservoir device into a perfect sink is given by

$$Q/t = [2\pi/\ln(R/r)]DC$$

where Q is the amount of drug released during the time interval t, R and r are the outer and the inner radius of the device, respectively, D is the diffusion constant, and C is the solubility of the drug in the polymer. The product DC is the permeability, expressed $\mu$g/cm.day, which is a material constant characterizing the release system for a specific polymer-drug combination.

Permeability data for levonorgestrel in combination with trimethylene carbonate-caprolactone copolymers of different compositions are presented in the last column of Table 2. These data demonstrate the improvement in drug release performance resulting from the incorporation of an increasing number of trimethylene carbonate units in the polycaprolactone chain. A trimethylene carbonate content slightly exceeding 10 mole=% already doubles the release performance of polycaprolactone and a fourfold increase over the polycaprolactone performance is obtained with a random copolymer containing about 22 mole-% trimethylene carbonate.

Inside the compositional range from zero to about 22 mole-% trimethylene carbonate content the permeabilities of the copolymers for levonorgestrel can be described by a second order parabola of the form

$$Permeability = 0.251 + 0.00113\ (TMC) + 0.00135\ (TMC)^2$$

where (TMC) represents the mole-% trimethylene carbonate units in the copolymer. Utilizing this equation, release rates of levonorgestrel in $\mu$g/cm.day can be calculated for cylindrical capsules possessing any outer diameter and wall thickness and being fabricated from random copolymers of trimethylene carbonate and caprolactone of any composition inside the range from zero to 20.0 mole-% trimethylene carbonate content. As an example, results of such calculations are presented in the attached Figure for capsules possessing an outer diameter of 2.4 mm and varying wall thickness.

The data of Table 2 demonstrate the surprisingly narrow compositional range suitable for the fabrication of implantable drug delivery devices possessing improved release properties. Up to 5% trimethylene carbonate content the permeability of a device increases by less than 10% over that of a device derived from polycaprolactone and even up to a trimethylene carbonate content of 10% the permeability increase of the device remains below 50%. Significant increases in permeability of more than 50% are observed with devices containing more than 10% trimethylene carbonate, and at 20 mole-% trimethylene carbonate content the copolymers exhibit three times the permeability of polycaprolactone. At the latter trimethylene carbonate content the crystalline melting point of the copolymer already approaches body temperature thus precluding to further increase the permeability of the devices by increasing their trimethylene carbonate contents.

TABLE 1

Effect of Trimethylene Carbonate Content on the Mechanical Properties of Random Copolymers of Caprolactone With Trimethylene Carbonate

| Copolymer Number | Mole-% Trimethylene Carbonate | Young's Modulus (MPa) | Yield Stress (MPa) | Nominal Stress at Break (MPa) | Draw Ratio at Break |
|---|---|---|---|---|---|
| PCL | 0.0 | 252 | 15.9 | 31.3 | 9.5 |
| COP-10 | 11.3 | 184 | 10.8 | 22.3 | 14.5 |
| COP-11 | 16.5 | 119 | 8.1 | 21.1 | 16.5 |
| COP-12 | 21.9 | 75 | 4.2 | 17.9 | 22.5 |

The preparation of a drug delivery device relating to this invention is performed by preparing a random copolymer of caprolactone and trimethylene carbonate of desired composition, followed by transforming said copolymer into tubular shape, and finally fabricating drug filled capsules from said copolymer tubing.

The copolyesters of this invention are random copolymers of trimethylene carbonate and caprolactone in the compositional range of 5-25 mole-% trimethylene carbonate, preferably inside the range of 10-20

## TABLE 2

Effect of Composition on Crystallinity, Crystalline Melting Temperature,
and Permeability for Levonorgestrel of Random Copolymers of Caprolactone
With Trimethylene Carbonate

| Copolymer Number | Mole-% Trimethylene Carbonate | Crystallinity Content (%) | Melting Temperature (°C) | Permeability ($\mu g/cm.day$) |
|---|---|---|---|---|
| PCL | 0.0 | 51.3 | 58.7 | 0.251 |
| COP-5 | 10.3 | 38.3 | 50.7 | --- |
| COP-10 | 11.3 | 36.3 | 47.3 | 0.449 |
| COP-6 | 15.6 | 32.3 | 46.3 | 0.559 |
| COP-11 | 16.5 | 26.4 | 45.7 | 0.680 |
| COP-7 | 18.7 | 29.2 | 44.3 | --- |
| COP-1 | 19.7 | 24.2 | 41.1 | 0.704 |
| COP-12 | 21.9 | 21.8 | 41.2 | 1.000 |
| COP-8 | 23.6 | 20.8 | 39.2 | --- |
| COP-2 | 30.1 | 13.1 | 37.0 | --- |

6

mole-% trimethylene carbonate. Caprolactone is a commercially available compound and trimethylene carbonate can be prepared as disclosed in the literature (W. H. Carothers and F. J. can Natta, J. Am. Chem. Soc., 52, 314 (1930) so that neither the compounds per se nor the method by which they are obtained constitutes any portion of the invention. The random copolymers are prepared by polymerizing a solution of trimethylene carbonate in caprolactone at elevated temperatures utilizing tin salts as catalysts in amounts of 100-500 ppm. The polymerization temperature can be in the range of 100-180°C, the preferred temperature range being 120-140°C. The tin salts preferentially used as polymerization catalysts are stannous octoate (2-ethyl-hexanoate) or stannous chloride although other tin salts can be used in amounts according to their catalytic activity. By utilizing carefully purified monomers and working under stringent exclusion of humidity high molecular weight random copolymers with intrinsic viscosities in chloroform exceeding 1.5 dl/g are obtained at better than 99% conversion as is well known to those skilled in the art. The transformation of the copolymer into tubular shape of the desired geometric dimension can be accomplished by conventional methods such as injection molding, dip casting, or extrusion utilizing screw or ram extruders.

During the last step of the process, the drug containing capsules are fabricated by filling required lengths of the copolymer tubing with the drug and subsequently heat sealing the tube ends. Preferentially, the drug is introduced in the form of a dry, micronized powder but mixtures of drug with a dispersing agent are also applicable. A dispersing agent of volatile nature may be added to the drug to facilitate the filling process, said dispersing agent being completely removed by evaporation prior to completely sealing the capsule. Non-volatile dispersing agents which remain in the sealed capsule together with the enclosed drug may also be used but under these conditions the permeability of the device may differ considerably from that of an identical device prepared in the absence of the dispersing agent. The kind and amount of drug, the dimension of the device, and the presence or absence of a dispersing agent may be chosen in accordance with the selected copolymer composition to provide optimal drug delivery conditions for the particular therapeutic application under consideration. Particularly, the composition of the random copolymer of caprolactone and trimethylene carbonate together with the geometric dimensions of the fabricated capsule will be decisive for the obtainable rate of the drug release.

The rate at which the polymeric device is degraded within the body is determined by the composition of the device as well as the mode of administration to the patient. Polycaprolactone has a halflife time of about nine months, i.e., during this time period the molecular weight of the polymer decreases to half its initial value. Copolymers with increasing trimethylene carbonate contents possess corresponding shorter halflife times.

The following examples are offered to more fully illustrate the invention, but are not to be construed as limiting the scope thereof.

EXAMPLE 1:

A solution of 3.3 g trimethylene carbonate in 15 g caprolactone and containing 50 ppm stannous octoate was charged to a glass vial equipped with a sealing capillary. The vial contents were degassed, the vial was sealed off, and then placed into a circulating air oven thermostated at 140°C. After 46 hours a completely colorless and high viscous polymer had formed which was removed with a spatula. The conversion was better than 99% according to the thermogravimetric analysis of the as-formed copolymer which indicated a weight loss of only 0.52% at 220°C. The intrinsic viscosity of the copolymer in chloroform at 30°C was 2.64 dl/g. The copolymer is designated COP-1 in Table 2.

A thin film, cast from chloroform solution, was rolled on a tubular Teflon support and then annealed at 80°C under vacuum. The resulting tube had an outer diameter of 2.276 mm and a wall thickness of 0.240 mm. Two capsules were prepared of 2.0 cm length which were filled with 16.7 and 11.1 mg of dry, micronized levonorgestrel. The capsules were kept submerged in 200 ml deionized water under mild shaking at 37.5°C. The water was exchanged three times a week and released drug was monitored spectrophotometrically at 240 nm utilizing a cell with 4 cm path length. Constant release rates of 18.9 and 18.6 μg/cm.day, respectively, were observed over a time period of 212 days.

A third copolymer capsule of the same dimensions as the previous ones and filled with 16.5 mg of dry, micronized levonorgestrel was implanted subcutaneously in the dorsal region of an adult female New Zealand white rabbit. Blood samples were collected three times per week and the serum level of levonorgestrel was determined by radioimmunoassay. During an observation period of 90 days of means serum level of levonorgestrel was 740 pg/ml with a standard error of 26 pg/ml.

EXAMPLE 2:

450 g caprolactone containing 200 ppm stannous octoate were charged to a stainless steel reactor consisting of a stainless steel tube closed with Teflon plugs carrying o-rings and being secured by, stainless steel caps. The reactor was kept in a circulating oven thermostated at 160°C for 24 hours. The polymer melt was extruded under nitrogen pressure in the form of a wire of about 3 mm diameter which was subsequently pelletized. According to thermal gravimetric analysis the conversion was better than 99.1%. The intrinsic viscosity of the polymer in chloroform was found to be 1.76 dl/g. Further data on this polymer are included in Tables 1 and 2 under the designation PCL. The pellets were fed to a screw extruder (Killion, Model KL-100) equipped with a tubing die. At a draw ratio of about 3:1 the final tube dimensions were 2.340 mm outer diameter with a wall thickness of 0.155 mm.

Two capsules of 2.5 cm length, loaded with 50.9 and 54.4 mg of dry, micronized levonorgestrel, and heat sealed on both ends were prepared for in vitro release studies. Utilizing deionized water as the release medium at 37.5°C, the release rate averages over a time period of 277 days were 11.4 and 10.8 $\mu$g/cm.day with standard errors of 0.21 and 0.34 $\mu$g/cm.day, respectively.

Two identically prepared capsules but loaded with 18.3 and 13.4 mg of dry, micronized levonorgestrel were implanted subcutaneously in the dorsal region of an adult female New Zealand white rabbit. Blood samples were collected three times per week and the serum level of levonorgestrel was determined by radioimmunoassay. During an observation period of 83 days the mean serum levels of levonorgestrel were 239 and 210 pg/ml with a standard errors of 11 and 13 pg/ml, respectively. These serum levels were only about one third of the serum level observed with the copolymer device described in Example 1 although the copolymer device had twice the wall thickness of the polycaprolactone devices.

EXAMPLE 3:

A copolymer was prepared according to Example 1 but utilizing 5.4 g trimethylene carbonate dissolved in 14 g caprolactone. The colorless copolymer had an intrinsic viscosity in chloroform of 1.45 dl/g. Both the melting point and the crystallinity content of the copolymer were too low for the fabrication of a drug release device. The copolymer is designated COP-2 in Table 2.

EXAMPLE 4:

A copolymer was prepared according to Example 1 but utilizing 1.03 g trimethylene carbonate dissolved in 10 g caprolactone. Thermogravimetric analysis revealed a weight loss of 0.89 at 220°C. The intrinsic viscosity of the copolymer in chloroform was 1.65 dl/g. The copolymer is designated COP-5 in Table 2.

A cylindrical capsule with 2.4 mm outer diameter and a wall thickness of 0.200 mm was prepared as in Example 1 and filled with dry, micronized norethindrone. The average in vitro release rate was found to be 46 $\mu$g/cm.day over a release period of 90 days.

EXAMPLE 5:

A copolymer utilizing 1.65 g trimethylene carbonate dissolved in 10 g caprolactone was prepared as described in Example 1. The conversion was better than 99.8% according to a weight loss of only 0.18 at 180°C. The intrinsic viscosity in chloroform was 1.74 dl/g. The copolymer is designated COP-6 in Table 2.

A cylindrical capsule of 2.3 cm length was prepared from a rolled film with an outer diameter of 2.139 mm and a wall thickness of 0.196 mm. The capsule was filled with 17.4 mg of dry, micronized levonorgestrel and sealed. The in vitro release rate averaged over 130 days was found to be 17.4 $\mu$g/cm.day with a standard error of 0.71 $\mu$g/cm.day.

EXAMPLE 6:

The procedure of Example 2 was repeated with incorporation of the following changes. The stainless steel reactor was charged with a solution of 51.9 g trimethylene carbonate dissolved in 454.6 g caprolactone and containing 500 ppm stannous octoate. The polymerization conditions were 120°C for 51 hours followed by 140°C for 16 hours. The conversion was better than 99.3% according to a weight loss of only 0.65% at 220°C. The intrinsic viscosity in chloroform was 1.73 dl/g. The copolymer is designated COP-10 in Tables 1 and 2.

Tubes were extruded with an outer diameter of 2.321 mm and a wall thickness of 0.209 mm which were used for the preparation of a capsule of 2.2 cm length to be filled with dry, micronized levonorgestrel and to be used in an in vitro release study at 37.5°C. Averaged over a time period of 90 days the release rate was found to be 14.2 $\mu$g/cm.day with a standard error of 0.51 $\mu$g/cm.day.

EXAMPLE 7:

A copolymer was prepared according to the procedure of Example 6 but utilizing a caprolactone solution composed of 75.0 g trimethylene carbonate in 425.6 g caprolactone. The conversion was better than 99.0% according to a weight loss of 0.97% at 220°C. The intrinsic viscosity in chloroform was 2.21 dl/g. The copolymer is designated COP-11 in Tables 1 and 2.

Two capsules were prepared of 2.5 cm length with an outer diameter of 2.630 mm and a wall thickness of 0.299 mm. The capsules, filled with 15.6 and 15.2 dry, micronized levonorgestrel exhibited in vitro release rates of 16.1 and 17.1 $\mu$g/cm.day with standard errors of 0.47 and 0.53 $\mu$g/cm.day, respectively, averaged over a time period of 90 days.

EXAMPLE 8:

A copolymer was prepared according to the procedure of Example 6 but utilizing a caprolactone solution composed of 102.5 g trimethylene carbonate in 409.0 g caprolactone. The conversion was better than 99.1% according to a weight loss of 0.83% at 220°C. The intrinsic viscosity in chloroform was 1.53 dl/g. The copolymer is designated COP-12 in Tables 1 and 2.

Two capsules were prepared of 2.7 cm length with an outer diameter of 2.452 mm and a wall thickness of 0.249 mm. The capsules, filled with 15.3 and 18.8 mg dry, micronized levonorgestrel exhibited in vitro release rates of 28.0 and 27.4 $\mu$g/cm.day with standard errors of 1.00 and 0.98 $\mu$g/cm.day, respectively, averaged over a time period of-90 days.

While the invention has been described above with reference to specific examples and embodiments, it will be understood that the invention is not to be limited to specific examples and embodiments except as defined in the following claims.

**Claims**
**Claims for the following Contracting States : AT, BE, CH, LI, DE, DK, FR, GB, IT, LU, NL, SE**

1. A biodegradable reservoir device for sustained delivery of a drug contained therein, said device comprising a tubular wall of a random copolymer comprising trimethylene carbonate and caprolactone, said trimethylene carbonate being present in amounts of 5-25 mole-%.

2. The biodegradable article of Claim 1, further comprising a non-hydrophilic drug contained therein.

3. The device of Claim 2, wherein said drug is selected from the group consisting of contraceptive agents, hormones,, narcotic antagonists, antineoplastic agents, anti-inflammatory agents, and mixtures thereof.

4. The article of Claim 3, wherein said drug comprises levonorgestrel.

5. The article of Claim 1 where said random copolymer comprises 10-20 mole-% trimethylene carbonate.

6. The article of Claim 1 where said random copolymer has an intrinsic viscosity in chloroform in excess of 1.5 dl/g.

7. The article of Claim 1 which is filled with the drug in the form of a dry, micronized powder.

8. The article of Claim 1 which is filled with the drug in the form of a powder mixed with a suitable diluent or dispersing agent which remains in the sealed capsule.

9. The article of Claim 1 which is filled with the drug in the form of a powder mixed with a volatile diluent, said diluent being removed by evaporation prior to finally sealing the capsule.

10. An implantable pharmaceutical composition comprising an intimate mixture of a drug with a random copolymer of caprolactone and trimethylene carbonate in the compositional range of 5-25 mole% trimethylene carbonate content, said pharmaceutical composition being in cylindrical or spherical shape.

11. A pharmaceutical composition of Claim 10 where said random copolymer is in the compositional range of 10-20 mole-% trimethylene carbonate content.

**Claims for the following Contracting States : ES, GR**

1. A biodegradable reservoir device for sustained delivery of a drug contained therein, said device comprising a tubular wall of a random copolymer comprising trimethylene carbonate and caprolactone, said trimethylene carbonate being present in amounts of 5-25 mole-%.

2. Process for the preparation of a biodegradable reservoir device for sustained delivery of a drug contained therein, comprising preparing a tubular wall of a random copolymer comprising trimethylene carbonate and caprolactone, said trimethylene carbonate being present in amounts of 5-25 mole-%, and introducing in the device a non hydrophilic drug.

3. The process of claim 2, wherein said drug is selected from the group consisting of contraceptive agents, hormones, narcotic antagonists, antineoplastic agents, anti-inflammatory agents, and mixtures thereof.

4. The process of claim 3 wherein said drug comprises levonorgestrel.

5. The process of claim 2 where said random copolymer comprises 10-20 mole-% trimethylene carbonate.

6. The process of claim 2 where said random copolymer has an intrinsic viscosity in chloroform in excess of 1.5 dl/g.

7. The process of claim 2 wherein the device is filled with the drug in the form of a dry, micronized powder.

8. The process of claim 2 wherein the device is filled with the drug in the form of a powder mixed with a suitable diluent or dispersing agent which remains in the sealed capsule.

9. The process of claim 2 wherein the device is filled with the drug in the form of a powder mixed with a volatile diluent, said diluent being removed by evaporation prior to finally sealing the capsule.

10. Process for the preparation of an implantable pharmaceutical composition comprising preparing an intimate mixture of a drug with random copolymer of caprolactone and trimethylene carbonate in the compositional range of 5-25 mole % trimethylene carbonate content, said pharmaceutical composition being in cylindrical or spherical shape.

11. The process of claim 10 where said random copolymer is in the compositional range of 10-20 mole-% trimethylene carbonate.

**Patentansprüche**

**Patentansprüche für folgende Vertragsstaaten : AT, BE, CH, LI, DE, DK, FR, GB, IT, LU, NL, SE**

1. Biologisch abbaubare Reservoirvorrichtung zur verzögerten Freigabe eines darin enthaltenen Arzneimittels, wobei diese Vorrichtung eine röhrenförmige Wand aus einem statistischen Copolymer, umfassend Trimethylencarbonat und Caprolacton, umfaßt, wobei das Trimethylencarbonat in Mengen von 5-25 Mol% vorhanden ist.

2. Biologisch abbaubarer Gegenstand nach Anspruch 1, weiterhin umfassend ein darin enthaltenes nichthydrophiles Arzneimittel.

**3.** Vorrichtung nach Anspruch 2, worin das Arzneimittel ausgewählt ist aus der Gruppe bestehend aus empfängnisverhütenden Mitteln, Hormonen, Betäubungsmittelantagonisten, antineoplastischen Mitteln, entzündungshemmenden Mitteln und Gemischen davon.

**4.** Gegenstand nach Anspruch 3, worin das Arzneimittel Levonorgestrel umfaßt.

**5.** Gegenstand nach Anspruch 1, worin das statistische Copolymer 10-20 Mol% Trimethylencarbonat umfaßt.

**6.** Gegenstand nach Anspruch 1, worin das statistische Copolymer eine innere Viskosität in Chloroform von mehr als 1,5 dl/g hat.

**7.** Gegenstand nach Anspruch 1, welcher mit dem Arzneimittel in Form eines trockenen mikronisierten Pulvers gefüllt ist.

**8.** Gegenstand nach Anspruch 1, welcher mit dem Arzneimittel in Form eines Pulvers, das mit einem geeigneten Verdünnungsmittel oder Dispergiermittel vermischt ist, welches in der verschlossenen Kapsel bleibt, gefüllt ist.

**9.** Gegenstand nach Anspruch 1, welcher mit dem Arzneimittel in Form eines Pulvers, das mit einem flüchtigen Verdünnungsmittel vermischt ist, gefüllt ist, wobei das Verdünnungsmittel durch Verdampfen vor dem endgültigen Verschließen der Kapsel entfernt wird.

**10.** Implantierbare pharmazeutische Zusammensetzung, umfassend ein inniges Gemisch aus einem Arzneimittel mit einem statistischen Copolymer von Caprolacton und Trimethylencarbonat im Zusammensetzungsbereich von 5-25 Mol% Trimethylencarbonat-Gehalt, wobei die pharmazeutische Zusammensetzung eine zylindrische oder kugelförmige Form besitzt.

**11.** Pharmazeutische Zusammensetzung nach Anspruch 10, worin das statistische Copolymer im Zusammensetzungsbereich von 10-20 Mol% Trimethylencarbonat-Gehalt ist.

**Patentansprüche für folgende Vertragsstaaten : ES, GR**

**1.** Biologisch abbaubare Reservoirvorrichtung zur verzögerten Freigabe eines darin enthaltenen Arzneimittels, wobei diese Vorrichtung eine röhrenförmige Wand aus einem statistischen Copolymer, umfassend Trimethylencarbonat und Caprolacton, umfaßt, wobei das Trimethylencarbonat in Mengen von 5-25 Mol% vorhanden ist.

**2.** Verfahren zur Herstellung einer biologisch abbaubaren Reservoirvorrichtung zur verzögerten Freigabe eines darin enthaltenen Arzneimittels, umfassend das Herstellen einer röhrenförmigen Wand aus einem statistischen Copolymer, umfassend Trimethylencarbonat und Caprolacton, wobei das Trimethylencarbonat in Mengen von 5-25 Mol% vorhanden ist, und das Einbringen eines nichthydrophilen Arzneimittels in die Vorrichtung.

**3.** Verfahren nach Anspruch 2, worin das Arzneimittel ausgewählt ist aus der Gruppe bestehend aus empfängnisverhütenden Mitteln, Hormonen, Betäubungsmittelantagonisten, antineoplastischen Mitteln, entzündungshemmenden Mitteln und Gemischen davon.

**4.** Verfahren nach Anspruch 3, worin das Arzneimittel Levonorgestrel umfaßt.

**5.** Verfahren nach Anspruch 2, worin das statistische Copolymer 10-20 Mol% Trimethylencarbonat umfaßt.

**6.** Verfahren nach Anspruch 2, worin das statistische Copolymer eine innere Viskosität in Chloroform von mehr als 1,5 dl/g hat.

**7.** Verfahren nach Anspruch 2, worin die Vorrichtung mit dem Arzneimittel in Form eines trockenen mikronisierten Pulvers gefüllt ist.

8. Verfahren nach Anspruch 2, worin die Vorrichtung mit dem Arzneimittel in Form eines Pulvers, das mit einem geeigneten Verdünnungsmittel oder Dispergiermittel vermischt ist, welches in der verschlossenen Kapsel bleibt, gefüllt ist.

9. Verfahren nach Anspruch 2, worin die Vorrichtung mit dem Arzneimittel in Form eines Pulvers, das mit einem flüchtigen Verdünnungsmittel vermischt ist, gefüllt ist, wobei das Verdünnungsmittel durch Verdampfen vor dem endgültigen Verschließen der Kapsel entfernt wird.

10. Verfahren zur Herstellung einer implantierbaren pharmazeutischen Zusammensetzung, umfassend das Herstellen eines innigen Gemisches aus einem Arzneimittel mit einem statistischen Copolymer von Caprolacton und Trimethylencarbonat im Zusammensetzungsbereich von 5-25 Mol% Trimethylencarbonat-Gehalt, wobei die pharmazeutische Zusammensetzung eine zylindrische oder kugelförmige Form besitzt.

11. Verfahren nach Anspruch 10, worin das statistische Copolymer im Zusammensetzungsbereich von 10-20 Mol% Trimethylencarbonat-Gehalt ist.

**Revendications**

**Revendication pour les Etats contractants suivants : AT, BE, CH, LI, DE, DK, FR, GB, IT, LU, NL, SE**

1. Dispositif réservoir biodégradable pour la délivrance prolongée d'un médicament qui y est contenu, ce dispositif comprenant une paroi tubulaire d'un copolymère statistique comprenant du carbonate de triméthylène et de la caprolactone, ce carbonate de triméthylène étant présent dans des proportions de 5 à 25 moles %.

2. Articles biodégradable selon la revendication 1, comprenant en outre un médicament non hydrophile contenu dedans.

3. Dispositif selon la revendication 2, dans lequel ce médicament est choisi parmi des agents contraceptifs, des hormones, des antagonistes des narcotiques, des agents antinéoplasiques, des agents anti-inflammatoires et des mélanges de ceux-ci.

4. Article selon la revendication 3, dans lequel ce médicament comprend du lévonorgestrel.

5. Article selon la revendication 1, dans lequel ce copolymère statistique comprend 10 à 20 moles % de carbonate de triméthylène.

6. Article selon la revendication 1, dans lequel ce copolymère statistique a une viscosité intrinsèque dans le chloroforme supérieure à 1,5 dl/g.

7. Article selon la revendication 1, qui est rempli du médicament sous la forme d'une poudre sèche, micronisée.

8. Article selon la revendication 1, qui est rempli du médicament sous la forme d'une poudre mélangée à un agent diluant ou dispersant approprié qui reste dans la capsule scellée.

9. Article selon la revendication 1, qui est rempli du médicament sous la forme d'une poudre mélangée à un agent diluant volatil, ce diluant étant éliminé par évaporation avant le scellement final de la capsule.

10. Composition pharmaceutique implantable comprenant un mélange intime d'un médicament avec un copolymère statistique de caprolactone et de carbonate de triméthylène dans l'intervalle de compositions de 5 à 25 moles % de carbonate de triméthylène, cette composition pharmaceutique étant de forme cylindrique ou sphérique.

11. Composition pharmaceutique selon la revendication 10, dans laquelle ce copolymère statistique est dans l'intervalle de compositions de 10 à 20 moles % de carbonate de triméthylène.

**Revendications pour les Etats contractants suivants : ES, GR**

1. Dispositif réservoir biodégradable pour la délivrance prolongée d'un médicament qui y est contenu, ce dispositif comprenant une paroi tubulaire d'un copolymère statistique comprenant du carbonate de triméthylène et de la caprolactone, ce carbonate de triméthylène étant présent dans des proportions de 5 à 25 moles %.

2. Procédé de préparation d'un dispositif réservoir biodégradable pour la délivrance prolongée d'un médicament qui y est contenu, comprenant la préparation d'une paroi tubulaire d'un copolymère statistique comprenant du carbonate de triméthylène et de la caprolactone, ce carbonate de triméthylène étant présent dans des proportions de 5 à 25 moles %, et l'introduction dans le dispositif d'un médicament non hydrophile.

3. Procédé selon la revendication 2, dans lequel ce médicament est choisi parmi des agents contraceptifs, des hormones, des antagonistes des narcotiques, des agents antinéoplasiques, des agents anti-inflammatoires et des mélanges de ceux-ci.

4. Procédé selon la revendication 3, dans lequel ce médicament comprend du lévonorgestrel.

5. Procédé selon la revendication 2, dans lequel ce copolymère statistique comprend 10 à 20 moles % de carbonate de triméthylène.

6. Procédé selon la revendication 2, dans lequel ce copolymère statistique a une viscosité intrinsèque dans le chloroforme supérieure à 1,5 dl/g.

7. Procédé selon la revendication 2, qui est rempli du médicament sous la forme d'une poudre sèche, micronisée.

8. Procédé selon la revendication 2, dans lequel le dispositif est rempli du médicament sous la forme d'une poudre mélangée à un agent diluant ou dispersant approprié qui reste dans la capsule scellée.

9. Procédé selon la revendication 2, dans lequel le dispositif est rempli du médicament sous la forme d'une poudre mélangée à un agent diluant volatil, ce diluant étant éliminé par évaporation avant le scellement final de la capsule.

10. Procédé de préparation d'une composition pharmaceutique implantable comprenant la préparation d'un mélange intime d'un médicament avec un copolymère statistique de caprolactone et de carbonate de triméthylène dans l'intervalle de compositions de 5 à 25 moles % de carbonate de triméthylène, cette composition pharmaceutique étant de forme cylindrique ou sphérique.

11. Procédé selon la revendication 10, dans laquelle ce copolymère statistique est dans l'intervalle de compositions de 10 à 20 moles % de carbonate de triméthylène.

WALL
THICKNESS

100μm

150μm

200μm

250μm

300μm

SPECIFIC
RELEASE
RATE
(μg/cm.DAY)

TRIMETHYLENE CARBONATE CONTENT (MOLE-%)

*FIG. 1*